# EUROPÄISCHE PATENTANMELDUNG

(11) **EP 0 732 316 A1**
(43) Veröffentlichungstag der Anmeldung: **18.09.1996**
(21) Anmeldenummer: 96103285.1
(22) Anmeldetag: 04.03.1996
(51) Int. Cl.: C07C 17/25

(54) **Verfahren zur katalytischen Dehydrohalogenierung von halogenierten Kohlenwasserstoffen**

(30) Priorität: 15.03.1995 DE 19509298
(71) Anmelder: BAYER AG, 51368 Leverkusen (DE)
(72) Erfinder: Hoeltzenbein, Peter, 41539 Dormagen (DE); Mottweiler, Renke, Dr., Houston, Texas 77059 (US); Baade, Wolfgang, Dr., 27793 Wildeshausen (DE)

(57) **Zusammenfassung**

Das erfindungsgemäße Verfahren zur katalytischen Dehydrohalogenierung von halogenierten Kohlenwasserstoffen wird durchgeführt, indem man die Dehydrohalogenierung in einstufiger Verfahrensweise in Gegenwart von wäßrigen Alkalilösungen bei Temperaturen von 10 bis 85°C, bei Drücken von 0,1 bis 2,5 bar und in Gegenwart von löslichen Phasen-Transfer-Katalysatoren unter Mischen durchführt, wobei die entstehenden, dehydrohalogenierten Kohlenwasserstoffe unmittelbar aus dem Reaktionsgemisch durch Abdampfen entfernt werden.

Nach dem erfindungsgemäßen Verfahren gelingt es, die Dehydrohalogenierung von halogenierten Kohlenwasserstoffen in energetisch günstiger Weise und ohne hohen apparativen Aufwand durchzuführen, wobei die dehydrohalogenierten Kohlenwasserstoffe in sehr guten Ausbeuten und hohen Reinheiten erhalten werden.

## Beschreibung

Die Erfindung betrifft ein verbessertes Verfahren zur katalytischen Dehydrohalogenierung von halogenierten Kohlenwasserstoffen in Gegenwart von Phasen-Transfer-Katalysatoren in wäßriger Alkalilösung.

Die katalytische Dehydrohalogenierung von halogenierten Kohlenwasserstoffen in wäßrig-alkalischem Medium in Gegenwart von Phasen-Transfer-Katalysatoren in mehrstufigen, gefluteten Rührkesselkaskaden ist seit langem bekannt (z.B. US 3.981.937).

In EP 0 110 377 wird ein verbessertes Verfahren zur katalytischen Dehydrohalogenierung von halogenierten Kohlenwasserstoffen beschrieben, demgemäß in einem mehrstufigen Verfahren die halogenierten Kohlenwasserstoffe und der Katalysator in den ersten Reaktor eindosiert werden, während frisches, wäßriges Alkali in den nachfolgenden Reaktor gegeben wird. Bei diesem Verfahren werden nach der Reaktion das entstandene dehydrohalogenierte Produkt und die salzhaltige, wäßrige Lösung mit dem Restalkali in einem zusätzlichen Schritt voneinander getrennt.

Nachteilig bei diesem Verfahren ist die lange Verweilzeit der erhaltenen dehydrohalogenierten Kohlenwasserstoffe im Reaktor, was zur Dimerisierung bzw. Polymerisierung der erhaltenen Produkte führt. Die Dimerisierung bzw. Polymerisierung der erhaltenen Produkte führt zu Ausbeuteverlusten. Darüber hinaus muß bei dem in EP 0 110 377 beschriebenen mehrstufigen Verfahren die entstehende Reaktionswärme gesondert durch Kühlung des Reaktors abgeführt werden, was erhöhte Energiekosten mit sich bringt. Ein weiterer Nachteil des in der genannten europäischen Patentanmeldung beschriebenen Verfahrens ist in der erzielten Ausbeute zu sehen, die noch verbesserungswürdig ist.

Das in der europäischen Patentanmeldung 0 114 643 beschriebene Verfahren zur Herstellung von Chloropren bezieht sich insbesondere auf die Ausgestaltung der Trennung von wäßriger und organischer Phase. Durch Einschalten eines Dekantierschrittes zwischen Reaktor und Dampfstripper kann der Dampfverbrauch verringert werden. Durch Absenken der Temperatur beim Dampfstrippen wird der Katalysator thermisch weniger zersetzt, so daß er teilweise wiederverwendet werden kann.

Nachteilig ist jedoch, daß sich durch zusätzliche Verweilzeiten im Reaktor der Abbau des dehydrohalogenierten Produkts erhöht (durch z.B. Dimerisierung, Polymerisierung), so daß die Ausbeute nicht befriedigend ist. Darüber hinaus muß die Reaktionswärme ebenfalls durch Kühlung abgeführt werden, was energetisch ungünstig ist. Außerdem ist der apparative Aufwand gegenüber dem aus US 3.981937 bekannten Verfahren erhöht.

Es wurde nun ein verbessertes Verfahren zur katalytischen Dehydrohalogenierung von halogenierten Kohlenwasserstoffen gefunden, das dadurch gekennzeichnet ist, daß man die Dehydrohalogenierung in einstufiger Verfahrensweise in Gegenwart von wäßrigen Alkalilösungen bei Temperaturen von 10 bis 85°C, bei Drücken von 0,05 bis 2,5 bar und in Gegenwart eines löslichen Phasen-Transfer-Katalysators durchführt, wobei die entstehenden dehydrohalogenierten Kohlenwasserstoffe unmittelbar durch Abdampfen unter Ausnutzung der Reaktionswärme aus dem Reaktor entfernt werden.

Als halogenierte Kohlenwasserstoffe, die in das erfindungsgemäße Verfahren eingesetzt werden, eignen sich insbesondere solche Kohlenwasserstoffe mit 3 bis 6 Kohlenstoffatomen, bevorzugt mit 4 bis 5, insbesondere 4 Kohlenstoffatomen. Die Kohlenwasserstoffe können mit bis zu 4 Halogenatomen, bevorzugt 2 bis 3 Halogenatomen, insbesondere Chlor, substituiert sein. Genannt werden bevorzugt 3,4-Dichlorbuten-1 und/oder 2,3,4-Trichlorbuten-1.

Als Phasen-Transfer-Katalysatoren können die für Dehydrohalogenierungsreaktionen bekannten Phasen-Transfer-Katalysatoren eingesetzt werden, insbesondere die quarternären Ammonium- und/oder Phosphoniumsalze. Wir verweisen in diesem Zusammenhang auf EP 0 110 377. Bevorzugt werden lösliche quarternäre Ammonium- und/oder Phosphoniumsalze in das erfindungsgemäße Verfahren eingesetzt, insbesondere N-Kokosfettsäure-N,N-bis-(2-hydroxyalkyl)-N-benzylammoniumchlorid.

Die Phasen-Transfer-Katalysatoren werden in Mengen von 0,15 bis 1,5 Gew.-%, bevorzugt 0,2 bis 0,4 Gew.-%, bezogen auf den eingesetzten chlorierten Kohlenwasserstoff, eingesetzt.

Als Alkalihydroxide können in das erfindungsgemäße Verfahren eingesetzt werden Natriumhydroxid, Kaliumhydroxid, Lithiumhydroxid, bevorzugt Natriumhydroxid. Die Alkalihydroxide werden in Form ihrer wäßrigen Lösung in das erfindungsgemäße Verfahren eingesetzt. Die Alkalihydroxide werden üblicherweise im molaren Überschuß, bezogen auf den eingesetzten Chlorkohlenwasserstoff, zugegeben. Im allgemeinen wird ein Überschuß von 2 bis 25 Mol-%, bevorzugt 5 bis 22 Mol-%, bezogen auf die Molzahl des eingesetzten Chlorkohlenwasserstoffs, eingesetzt.

Die Konzentration der wäßrigen Alkali- und/oder Erdalkalilösung liegt üblicherweise bei 10 bis 25 Gew.-%, bevorzugt bei 5 bis 22 Gew.-%.

Wie erwähnt, wird das erfindungsgemäße Verfahren in einstufiger Reaktionsführung unter innigem Mischen der Komponenten durchgeführt. Die Temperaturen, bei denen die Reaktion durchgeführt wird, liegen bevorzugt bei 20 bis 75°C, die Drücke bevorzugt bei 0,2 bis 2 bar.

Das erfindungsgemäße Verfahren kann sowohl kontinuierlich als auch halbkontinuierlich (Zulaufverfahren) durchgeführt werden.

Für das erfindungsgemäße Verfahren ist es wesentlich, daß die bei der Reaktion entstehenden, dehydrohalogenierten Kohlenwasserstoffe, unmittelbar aus dem Reaktionsgemisch entfernt werden. Im allgemeinen liegt der Siedepunkt der dehydrohalogenierten Produkte, wie 2-Chlorbutadien-1,3 oder 2,3-Dichlorbutadien-1,3, unter dem Siedepunkt der Ausgangsprodukte, so daß die Reaktionswärme als Energie für das Abdestillieren direkt benutzt werden kann.

Beim Abdampfen des entstandenen Produktes werden bis zu 3 % des Ausgangsproduktes mitverdampft, so daß eine direkte Rektifikation der aufsteigenden Brüden den nicht dehydrohalogenierten Kohlenwasserstoff in die Reaktionszone zurückbringt. Dadurch erhalt man sowohl einen quantitativen Umsatz der Ausgangsprodukte als auch eine sehr hohe Reinheit des Zielproduktes. Das erfindungsgemäße Verfahren kann im allgemeinen wie folgt durchgeführt werden:

In einen Rührkesselreaktor werden die wäßrige Alkalilauge, Dichlorbuten und der Phasentransferkatalysator eindosiert und das Gemisch reagiert unter intensivem Rühren bei einer Temperatur von ca. 65 bis 70°C. Das entstehende Chloropren wird sofort aus dem Reaktionsgemisch durch Abdampfen entfernt und die zurückbleibende wäßrige alkalische, salzhaltige Phase aus dem Reaktor entnommen. Für das Verfahren ist es vorteilhaft, wenn die das Chloropren enthaltenden, aufsteigenden Brüden einer direkten Rektifikation zur Produktabtrennung unterworfen werden.

Nach dem erfindungsgemäßen Verfahren werden die unmittelbar nach der Dehydrohalogenierungsreaktion erhaltenen Produkte in einer außerordentlich hohen Reinheit erhalten (>99,7 %). Die Ausbeute der nach dem erfindungsgemäßen Verfahren erhaltenen Produkte ist praktisch quantitativ.

Gegenüber dem Stand der Technik bietet das erfindungsgemäße Verfahren folgende Vorteile: erhebliche Energieeinsparung durch geringere Menge an benötigtem Dampf und Kühlwasser, Rückstandsreduzierung durch praktisch vollständigen Umsatz und Vermeidung von Neben- und Folgereaktionen (Di- bzw. Polymerisierung), Abwasserreduzierung durch geringen Dampfeinsatz, Reduzierung des apparativen Aufwandes sowie hohe Produktreinheit.

Die nachfolgenden Beispiele sollen das erfindungsgemäße Verfahren verdeutlichen:

### Beispiele

### Beispiel 1

In einen kontinuierlich betriebenen 1 l-Rührkesselreaktor werden 666 ml/h 20 %ige Natronlauge, 340 ml/h Dichlorbuten, 4000 ppm N-Kokosfettsäure-N,N-bis(2-hydroxypropyl)-N-benzyl-ammoniumchlorid (Phasentransferkatalysator) und 50 ppm Phenotriazin eingefahren. Der gut durchmischte Reaktor wird standardmäßig bei einer Verweilzeit von 20 Minuten betrieben. Die Reaktionstemperatur wird isotherm auf 65°C gehalten. Das gebildete Chloropren wird sofort über eine Füllkörperkolonne (Länge 30 cm, Durchmesser 2,5 cm, mit 0,6 cm Raschigringen gefüllt), die mit einem Rücklaufteiler ausgerüstet ist, abgezogen. Das Rücklaufverhältnis wurde auf 1:3 eingestellt. Der Reaktor wird standgeregelt und die zurückbleibende wäßrige alkalische, salzhaltige Phase aus dem Reaktor herausgefahren. Die über Kopf entnommene Fraktion enthält 99,6 % 2-Chloropren, 0,2 % 1-Chloropren und <100 ppm Dichlorbuten. In der Wasserphase werden <50 ppm Chloropren und <10 ppm Dichlorbuten gefunden. Insgesamt beträgt der Anteil an gebildeten Dimeren und Oligomeren weniger als 500 ppm. Die Konzentrationen wurden gaschromatographisch ermittelt. Eine Kontaktkühlung des Reaktors war nicht notwendig.

### Beispiel 2

Beispiel 1 wird wiederholt, wobei das Rücklaufverhältnis auf 1:2 eingestellt wurde. Die Reaktorinnentemperatur betrug 64°C. Die Kopffraktion enthielt 99,7 % 2-Chloropren, 0,2 % 1-Chloropren und <50 ppm Dichlorbuten. In der Wasserphase wurden weniger als 30 ppm Chloropren und <0,5 ppm Dichlorbuten gefunden. Insgesamt wurden weniger als 300 ppm Dimere und Oligomere gemessen. Eine Kontaktkühlung des Reaktors war ebenfalls nicht notwendig.

### Beispiel 3

Beispiel 2 wird wiederholt, wobei statt dem quaternären Ammoniumsalz das entsprechende quaternäre Phosphoniumsalz als Phasen-Transfer-Katalysator eingesetzt wurde. Die Reaktorinnentemperatur betrug 69°C. Die ansonsten erhaltenen Ergebnisse blieben unverändert.

### Beispiel 4

In einem 1 l-Rührkesselreaktor werden 630 ml 20%-ige Natronlauge und 300 ml dest. Wasser vorgelegt. Der Reaktor wird auf 30°C erwärmt und ein Vakuum von 50 mbar angelegt. Dann werden innerhalb von 90 Minuten 500 ml Trichlorbuten und 2 000 ppm N-Kokosfettsäure-N,N-bis(2-hydroxypropyl)-N-benzyl-ammoniumchlorid (Phasentransferkatalysator) zudosiert und anschließend weitere 20 Minuten nachgerührt. Der Reaktor wird währenddessen bei einem Druck von ≦ 50 mbar und isotherm bei 29°C gehalten. Das gebildete Dichlorbutadien wird sofort über eine Füllkörperkolonne (Länge 30 cm, Durchmesser 25 mm, mit 6 mm Raschigringen gefüllt), die mit einem Rücklaufteiler ausgerüstet ist, abgezogen. Das Rücklaufverhältnis wurde auf 2:1 eingestellt. Die über Kopf entnommene Fraktion enthält 99,1 % Dichlorbutadien und 0,3 % Trichlorbuten. Die Konzentrationen wurden gaschromatographisch ermittelt. Eine Kontaktkühlung des Reaktors war nicht notwendig.

## Patentansprüche

1. Verfahren zur katalytischen Dehydrohalogenierung von halogenierten Kohlenwasserstoffen, dadurch gekennzeichnet, daß man die Dehydrohalogenierung in einstufiger Verfahrensweise in Gegenwart von wäßrigen Alkalilösungen bei Temperaturen von 10 bis 85°C, bei Drücken von 0,1 bis 2,5 bar und in Gegenwart von löslichen Phasen-Transfer-Katalysatoren unter Mischen durchführt, wobei die entstehenden, dehydrohalogenierten Kohlenwasserstoffe unmittelbar aus dem Reaktionsgemisch durch Abdampfen entfernt werden.

2. Verfahren nach Anspruch 1, dadurch gekennzeichnet, daß man die dehydrohalogenierten Kohlenwasserstoffe enthaltenden aufsteigenden Brüden einer direkten Rektifikation unterwirft.

3. Verfahren nach Ansprüchen 1 und 2, dadurch gekennzeichnet, daß man das Verfahren bei Temperaturen von 20 bis 75°C und bei Drücken von 0,2 bis 2 bar durchführt.
